# EUROPÄISCHE PATENTSCHRIFT

(11) **EP 0 779 280 B1**
(45) Veröffentlichungstag und Bekanntmachung des Hinweises auf die Patenterteilung: **27.02.2002**
(21) Anmeldenummer: 96810845.6
(22) Anmeldetag: 03.12.1996
(51) Int. Cl.: C07D 251/24

(54) **Verfahren zur Herstellung von 2-(2,4-Dihydroxyphenyl)-4,6-bis-(2,4-dimethylphenyl)-s-triazin**
Process for the preparation of 2-(2,4-dihydroxyphenyl)-4,6-bis-(2,-dimethylphenyl)-s-triazine
Procédé pour la préparation de 2-(2,4-Dihydroxyphenyl)-4,6-bis-(2,4-dimethylphenyl)-s-triazine

(30) Priorität: 14.12.1995 CH 354195
(43) Veröffentlichungstag der Anmeldung: 18.06.1997
(73) Patentinhaber: Ciba Specialty Chemicals Holding Inc., 4057 Basel (CH)
(72) Erfinder: Orban, Ivan, 4052 Basel (CH); Holer, Martin, 4312 Magden (CH); Kaufmann, André, 4432 Lampenberg (CH)

(56) Entgegenhaltungen:
- DE-B- 1 169 947
- US-A- 3 244 708
- US-A- 3 268 474
- HELVETICA CHIMICA ACTA, Bd. 55, Nr. 5, 1972, Seiten 1566-1595, XP000651570 H. BRUNETTI; C.E. LÜTHI: "Die Synthese von asymmetrisch substituierten o-Hydoxyphenyl-s-triazinen"

## Beschreibung

Die vorliegende Erfindung betrifft ein neues, vereinfachtes und damit wirtschaftlicheres Verfahren zur Herstellung von 2-(2,4-Dihydroxyphenyl)-4,6-bis-(2,4-dimethylphenyl)-s-triazin aus Cyanurchlorid.

Die Umsetzung von Cyanurchlorid mit 1,3-Xylol unter Friedel-Crafts-Bedingungen zu 2-Chloro-4,6-bis-(2,4-dimethylphenyl)-s-triazin ist bekannt, z. B. aus der DE-A-1 169 947 und Helv. Chim. Acta 55, 1589 (1972). Das Produkt kann aber auch mit einem entsprechenden Grignard-Reagenz erhalten werden, wie z. B. beschrieben in Helv. Chim. Acta 33, 1365 (1950) und US-A-4 092 466.
Die weitere Umsetzung des zuvor durch Umkristallisation gereinigten 2-Chloro-4,6-bis-(2,4-dimethylphenyl)-s-triazins mit Resorcin ist beschrieben in US-A-3 244 708.

Die direkte Umsetzung von Cyanurchlorid mit zwei Äquivalenten 1,3-Xylol hat jedoch den Nachteil, dass mono- und tris-substituierte Produkte mitentstehen. Vor allem das Mono-Xylyl-Derivat ist unerwünscht, da es bei der Folgestufe mit Resorcin zur entsprechenden Bis-Resorcinol-Verbindung führt. Letztere kann beim Einsatz der aus der Verbindung der Formel I hergestellten UV-Absorber in verschiedenen Polymersubstraten, z. B. in bestimmten Lacken, zu Vergilbungen führen. Die Bildung dieser Bis-Resorcinol-Verbindung wurde früher über den Umweg des Dichlor-alkoxy-s-trazins ausgeschlossen [Helv. Chim. Acta 55, 1575 (1972)].

Es wurde nun gefunden, dass 2-(2,4-Dihydroxyphenyl)-4,6-bis-(2,4-dimethylphenyl)-s-triazin durch Umsetzung von Cyanurchlorid mit 1,3-Xylol in Gegenwart einer Lewis-Säure und eines inerten chlorierten aromatischen Lösungsmittels ohne Isolierung der Chlor-s-triazin-Zwischenstufe durch anschliessende Umsetzung mit Resorcin im sogenannten EintopfVerfahren hergestellt werden kann; dabei entsteht im ersten Reaktionsschritt überraschenderweise ohne Erhöhung des Tris-Anteils praktisch keine monoaryl-substituierte Dichlor-s-Triazin-Verbindung.

Weiterhin konnte durch die Reaktionsführung eine unkontrollierte Exothermie, welche beim gleichzeitigen Vorlegen der Edukte und des Katalysators [siehe dazu DE-A-1 169 947 bzw. BP 884 802] auftreten kann und das damit verbundene Sicherheitsrisiko ausgeschlossen werden.

Als Lösungsmittel wird weder die Verbindung mit der Formel III selbst noch Schwefelkohlenstoff [Helv. Chim. Acta 55, 1589 (1972)] verwendet, und das Reaktionsgemisch wird auch nicht fest, wie es in der DE-A-1 169 947 beschrieben ist.

Gegenstand der vorliegenden Erfindung ist daher ein Verfahren zur Herstellung von 2-(2,4-Dihydroxyphenyl)-4,6-bis-(2,4-dimethylphenyl)-s-triazin der Formel I 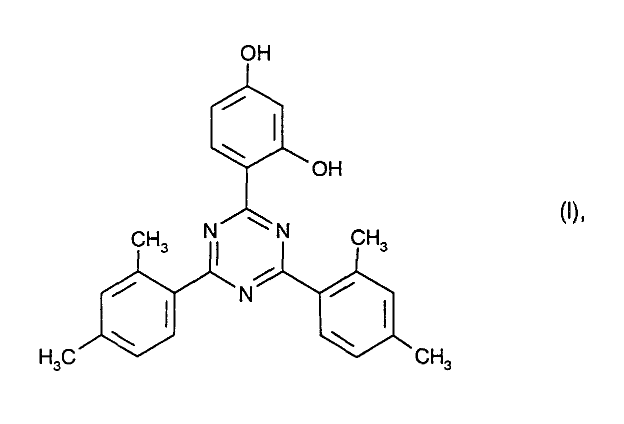 dadurch gekennzeichnet, daß man
a) Cyanurchlorid der Formel II 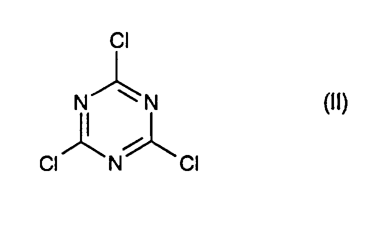 bei einer Temperatur von 0 bis 110°C in Gegenwart einer Lewis-Säure bei einem Verhältnis von 2,3 bis 3,0 Mol Lewis-Säure pro Mol eingesetztem Cyanurchlorid in Anwesenheit mindestens eines inerten chlorierten aromatischen Lösungsmittels mit 1,3-Xylol der Formel III 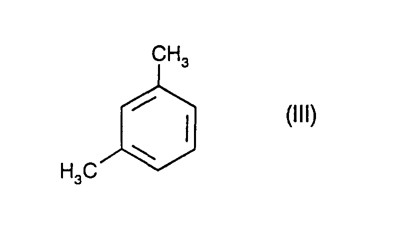 bei einem Verhältnis von 2,1 bis 2,5 Mol Verbindung der Formel III pro Mol eingesetztem Cyanurchlorid unter Zugabe von Verbindung der Formel III in einem Zeitraum von 5 bis 30 Stunden zur Verbindung der Formel IV 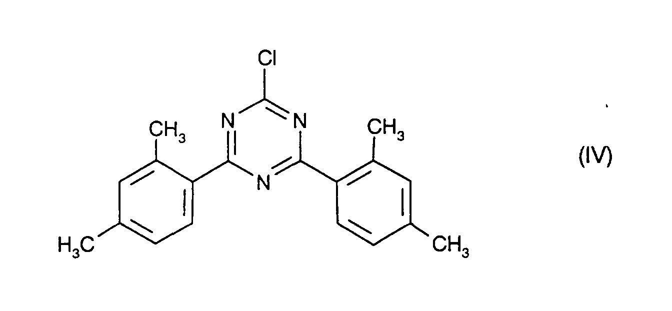 umsetzt und anschliessend
b) ohne Isolierung der erhaltenen Verbindung der Formel IV diese mit Resorcin bei einem Verhältnis von 0,5 bis 1,0 Mol Resorcin pro Mol eingesetztem Cyanurchlorid bei einer Temperatur von 0 bis 100°C zur Verbindung der Formel I umsetzt.

Die Reaktionsführung im Verfahrensschritt a) geschieht bevorzugt so, dass man die Verbindung der Formel III in 2 Portionen zugibt. Zweckmässigerweise werden zunächst 0,5 bis 1,1 Mol und anschliessend 2,0 bis 1,0 Mol der Verbindung mit der Formel III zugegeben. Die Zugabe der ersten Portion erfolgt bei 60 bis 110°C, insbesondere 75 bis 95°C, jene der zweiten bei 20 bis 50°C, insbesondere 30 bis 40°C. Besonders zweckmässig werden zunächst 0,5 bis 1,1 Mol der Verbindung mit der Formel III bei 60 bis 110°C zur Verbindung mit der Formel II in einem Zeitraum von 2 bis 12 Stunden hinzugegeben, während die zweite Portion der Verbindung mit der Formel III 2,0 bis 1,0 Mol beträgt und bei 20 bis 50°C in einem Zeitraum von 5 bis 15 Stunden zudosiert wird. Die Abkühlphase zwischen den beiden Temperaturbereichen hat dabei eine Zeitdauer von ≤ 3 Stunden, beispielsweise 0,1 bis 3 Stunden, insbesondere 0,5 bis 2,5 Stunden.
Besonders bevorzugt wird die Reaktionsführung im Verfahrensschritt a) so ausgeführt, dass die Zugabe von 0,85 bis 1,0 Mol der Verbindung mit der Formel III innerhalb von 3,5 bis 4,5 Stunden bei 83 bis 87°C erfolgt, anschliessend während 1,4 bis 1,6 Stunden auf 33 bis 37°C abgekühlt wird, worauf die Zugabe von 1,45 bis 1,3 Mol der Verbindung mit der Formel III innerhalb von 5,5 bis 6,5 Stunden im Temperaturbereich von 33 bis 37°C erfolgt.

In der Reaktionsstufe a) wird ein aromatisches Lösungsmittel oder ein Gemisch eingesetzt. Beispiele hierfür sind Mono-, Di- oder Trichlorbenzol oder Mischungen davon, insbesondere Chlorbenzol oder Dichlorbenzol, z. B. 1,2-Dichlorbenzol.
In der zweiten Reaktionsstufe b) kann ein von der ersten Reaktionsstufe a) verschiedenes Lösungsmittel verwendet werden.
Als Lösungsmittel kommen beispielsweise in Betracht: Nitrobenzol, Anisol, Chlorbenzol und 1,2-Dichlorbenzol oder Mischungen dieser untereinander.
Wird in der zweiten Reaktionsstufe b) ein anderes Lösungmittel verwendet, so kann das erste Lösungsmittel partiell oder ganz z. B. durch Abdestillieren entfernt werden.

Bervorzugt wird jedoch auch in Reaktionsstufe b) ein chloriertes aromatisches Lösungsmittel eingesetzt, insbesondere das gleiche wie in Reaktionsstufe a).

In Reaktionsstufe a) wird als Lewis-Säure in der dem Fachmann als Friedel-Crafts-Reaktion bekannten Umsetzung vorzugsweise Aluminiumchlorid oder Aluminiumbromid verwendet, wobei ersteres besonders zu bevorzugen ist. Die genannten Lewis-Säuren sind zweckmässig im wesentlichen wasserfrei.
Die Lewis-Säure wird bevorzugt in einem Verhältnis von 2,4 bis 2,6 Mol pro Mol Cyanurchlorid eingesetzt.

Der Verfahrensschritt b) kann sich zweckmässigerweise ohne Isolierung des im ersten Verfahrensschritt a) entstandenen Produktes mit der Formel IV direkt anschliessen, d. h. eine Aufarbeitung ist bei den hier genannten Reaktionsbedingungen nicht notwendig, da im Verfahrensschritt a) praktisch keine Mono-Xylyl-Verbindung entsteht. Der relativ hohe prozentuale Anteil der Tris-Xylyl-Verbindung stört den weiteren Reaktionsverlauf nicht, da diese Verbindung bei der Isolierung des gewünschten Produktes in Lösung bleibt und sich daher im Filtrat wiederfindet.
Im Verfahrensschritt b) werden bevorzugt 0,7 bis 0,8 Mol Resorcin pro Mol Cyanurchlorid eingesetzt und die Temperatur wird während der Reaktion zweckmässig kontinuierlich von 35 auf 80°C erhöht.
Die Isolierung des Endproduktes kann nach an sich üblichen Verfahren erfolgen. Zweckmässig wird zunächst die Lewis-Säure hydrolysiert.
Wird beispielsweise ein chloriertes aromatisches Lösungsmittel verwendet, kann durch langsame Zugabe eines apolaren Lösungsmittels und Animpfen die Verbindung der Formel I als kristalline Verbindung erhalten werden.
Zweckmässigerweise wird die Temperatur während der Kristallisation gesenkt und das erhaltene Produkt vorzugsweise mit einer Nutsche isoliert.
Das vom Lösungsmittel abgetrennte Produkt wird zweckmässigerweise auf der Nutsche gewaschen und anschliessend getrocknet, wobei das Trocknen vorzugsweise im Vakuum bei 65 bis 70°C erfolgt.

Die Verbindung der Formel I zeichnet sich selbst bereits durch ihre lichtstabiliserende Wirkung in organischen Materialien aus; sie lässt sich darüberhinaus vorteilhaft durch Substitution des Wasserstoffs der p-ständigen Hydroxy-Funktion zur Herstellung von Verbindungen wie 2-(2-Hydroxy-4-alkoxyphenyl)-4,6-bis-(2,4-dimethylphenyl)-s-triazinen einsetzen, die als Lichstabilisatoren für organisches Material, insbesondere synthetische Polymere, bekannt sind. Beispiele für diese Verwendungen von Verbindungen ähnlich der Formel I finden sich beispielsweise in der US-A-3 268 474.

Die vorliegende Erfindung betrifft auch ein Verfahren zur Herstellung des im vorstehend beschriebenen Verfahren auftretenden Zwischenproduktes der Formel IV 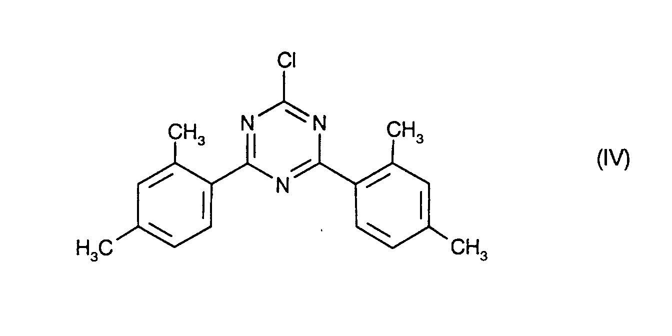 dadurch gekennzeichnet, dass man Cyanurchlorid der Formel II 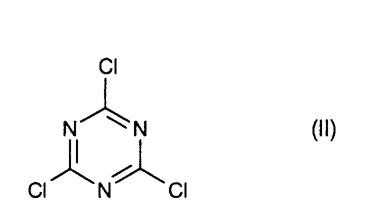 bei einer Temperatur von 0 bis 110°C in Gegenwart einer Lewis-Säure bei einem Verhältnis von 2,3 bis 3,0 Mol Lewis-Säure pro Mol eingesetztem Cyanurchlorid in Anwesenheit mindestens eines inerten chlorierten aromatischen Lösungsmittels mit 1,3-Xylol der Formel III 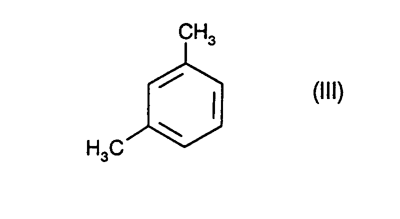 bei einem Verhältnis von 2,1 bis 2,5 Mol Verbindung der Formel III pro Mol eingesetztem Cyanurchlorid unter Zugabe von Verbindung der Formel III in einem Zeitraum von 5 bis 30 Stunden zur Verbindung der Formel IV umsetzt und die erhaltene Verbindung der Formel IV isoliert.

Die Verfahrensmassnahmen und die bevorzugten Verfahrensausgestaltungen entsprechen denen der vorstehend für die Reaktionsstufe a) des erfindungsgemässen Eintopfverfahrens beschriebenen. Die Isolierung der Verbindung der Formel IV erfolgt nach dem Fachmann bekannten Verfahren, wie z. B. Destillation des Lösungsmittels, Kristallisation durch Zugabe eines geeigneten Lösungsmittels u.s.w..

Das nachfolgende Beispiel illustriert das erfindungsgemässe Verfahren weiter. Alle Angaben in Prozenten beziehen sich auf das Gewicht, soweit nichts anderes angegeben ist.

### Beispiel: Herstellung von 2-(2,4-Dihydroxyphenyl)-4,6-bis-(2,4-dimethylphenyl)-s-triazin

### Verfahrensschritt a)

Zu 110,7 g (0,60 Mol) Cyanurchlorid, gelöst in 360 g 1,2-Dichlorbenzol, werden unter Rühren (ca. 200 UpM) 200 g (1,50 Mol) Aluminiumchlorid gegeben. Anschliessend wird die gut rührbare Suspension innerhalb von 30 Minuten auf 84 bis 86°C erwärmt und bei dieser Temperatur 15 Minuten lang gehalten. Bei ca. 200 UpM werden 67,9 ml (58,6 g, 0,55 Mol) m-Xylol während 4 Stunden bei 84 bis 86°C zudosiert (exotherme Reaktion!), anschliessend wird die Suspension innerhalb von 1,5 Stunden möglichst gleichmässig auf 34 bis 36°C abgekühlt.
Die Zugabe von weiteren 101,8 ml (87,95 g, 0,83 Mol) m-Xylol erfolgt bei 34 bis 36°C (exotherme Reaktion!) in einem Zeitraum von 6 Stunden. Kurz vor Zulaufende wird wenig HCI-Gas frei, das mittels NaOH (40%-ig) neutralisiert wird. Nach beendeter Xylol-Dosierung wird das gut rührbare dunkelbraune Reaktionsgemisch noch 15 Minuten bei 34 bis 36°C weitergerührt.

### Verfahrensschritt b)

Zu der obigen Reaktionslösung werden 9,9 g (0,09 Mol) Resorcin innerhalb 1 Stunde zudosiert, die Temperatur beträgt weiterhin 34 bis 36°C. Die nächsten 9,9 g Resorcin werden ebenfalls innerhalb 1 Stunde hinzugegeben, währenddessen wird die Temperatur von 35 auf 48°C erhöht. Es werden bei 48°C innerhalb 3 Stunden weitere 29,7 g Resorcin (0,45 Mol) hinzugeben.
Anschliessend wird das Reaktionsgemisch im Zeitraum von 3 Stunden auf 79 bis 81°C aufgeheizt und bei dieser Temperatur 1 Stunde lang weitergerührt. Das über die gesamte Reaktionsdauer freiwerdende HCI-Gas wird mittels 40%iger Natronlauge neutralisiert.

Zur Aufarbeitung wird die obige warme Reaktionslösung zu einer bei 300 bis 350 UpM gerührten Lösung von 750 g Wasser und 750 g Methylisobutylketon oder Methylethylketon, die sich bei Raumtemperatur befindet, innerhalb von 10 bis 15 Minuten gegeben. Bei 68 bis 70°C wird 10 Minuten lang ausgerührt. Nach der Phasentrennung wird die untere (wässrige) Phase abgetrennt und die obere organische Phase mit 375 g 3%iger Salzsäure versetzt. Es wird nochmals bei 68 bis 70°C 10 Minuten lang gerührt und nach erfolgter Phasentrennung die untere (wässrige) Phase entfernt.
Aus der organischen Phase werden bei Temperaturen von ca. 104 bis 130°C und anfänglichem Normaldruck bis 150 - 155 mbar am Schluss die Lösungsmittel entfernt.
Zum Destillationsrückstand gibt man 400 g 1,2-Dichlorbenzol so hinzu, dass die Temperatur nicht unter 120°C sinkt. Anschliessend werden 580 g Heptan-lsomerengemisch innerhalb 1 Stunde so zudosiert, dass immer ein deutlicher Rückfluss herrscht. Nachdem ca. 200 g Heptan zugegeben sind, wird die Lösung laufend geimpft [1 g 2-(2,4-Dihydroxyphenyl)-4,6-bis-(2,4-dimethylphenyl)-s-triazin in 20 g Heptan]. Nachdem etwa 50 % des Heptans zudosiert worden sind, beginnt das Produkt langsam in feinen Nadeln zu kristallisieren. Nach beendeter Heptan-Zugabe wird die dickflüssige, aber gut rührbare Suspension noch 1 Stunde am Rückfluss (110 bis 112°C) gehalten.
Nachdem man die Suspension auf 20 bis 22°C abgekühlt hat, wird diese abgenutscht und das Nutschgut zuerst mit 500 g 1,2-Dichlorbenzol/Heptan (1:1) und anschliessend mit 400 g Methanol gewaschen.
Das feuchte Nutschgut (ca. 300 g) wird im Vakuum bei 65 bis 70°C getrocknet.

Ausbeute: 146,5 g [ca. 98,5% (LC)] ⇒ **144,3 g** [100 %] = **60,5 %** der Theorie, bezogen auf Cyanurchlorid

## Patentansprüche

1. Verfahren zur Herstellung von 2-(2,4-Dihydroxyphenyl)-4,6-bis-(2,4-dimethylphenyl)-s-triazin der Formel I 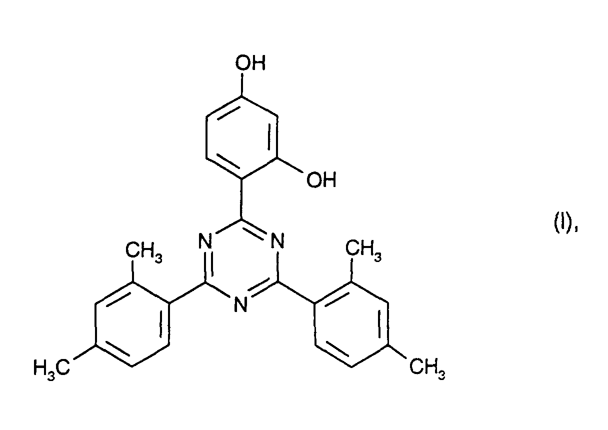 **dadurch gekennzeichnet, daß** man
a) Cyanurchlorid der Formel II 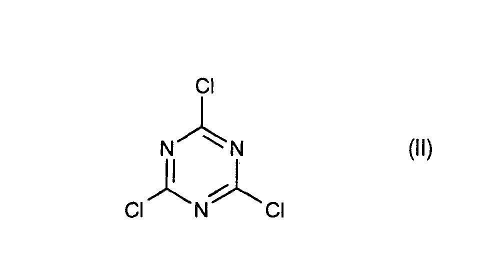 bei einer Temperatur von 0 bis 110°C in Gegenwart einer Lewis-Säure bei einem Verhältnis von 2,3 bis 3,0 Mol Lewis-Säure pro Mol eingesetztem Cyanurchlorid in Anwesenheit mindestens eines inerten chlorierten aromatischen Lösungsmittels mit 1,3-Xylol der Formel III 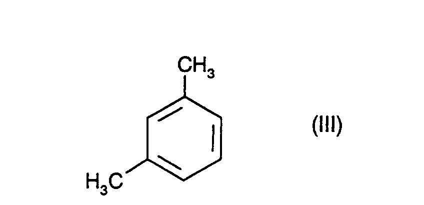 bei einem Verhältnis von 2,1 bis 2,5 Mol Verbindung der Formel III pro Mol eingesetztem Cyanurchlorid unter Zugabe von Verbindung der Formel III in einem Zeitraum von 5 bis 30 Stunden zur Verbindung der Formel IV 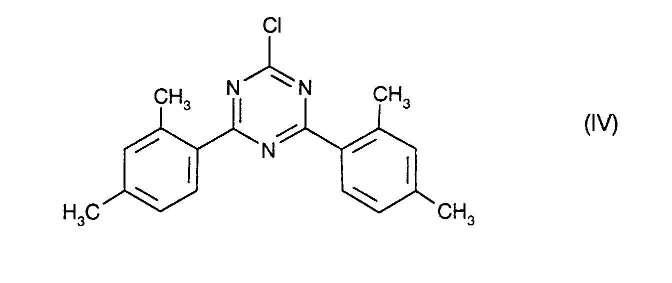 umsetzt und anschliessend
b) ohne Isolierung der erhaltenen Verbindung der Formel IV diese mit Resorcin bei einem Verhältnis von 0,5 bis 1,0 Mol Resorcin pro Mol eingesetztem Cyanurchlorid bei einer Temperatur von 0 bis 100°C zur Verbindung der Formel I umsetzt.

2. Verfahren gemäss Anspruch 1, worin in Verfahrensschritt a) die Verbindung der Formel III in 2 Portionen zugegeben wird.

3. Verfahren gemäss Anspruch 2, worin zunächst 0,5 bis 1,1 Mol und anschliessend 2,0 bis 1,0 Mol der Verbindung mit der Formel III zugegeben werden.

4. Verfahren gemäss Anspruch 2, worin die Zugabe der ersten Portion bei 60 bis 110°C, jene der zweiten bei 20 bis 50°C erfolgt.

5. Verfahren gemäss Anspruch 1, worin in Verfahrensschritt a) die Zugabe von 0,5 bis 1,1 Mol der Verbindung mit der Formel III innerhalb von 2 bis 12 Stunden bei 60 bis 110°C erfolgt, anschliessend innerhalb von 0 bis 3 Stunden auf 20 bis 50°C abgekühlt wird, worauf die Zugabe von 2,0 bis 1,0 Mol der Verbindung mit der Formel III innerhalb von 5 bis 15 Stunden im Temperaturbereich von 20 bis 50°C erfolgt.

6. Verfahren gemäss Anspruch 1, worin in Verfahrensschritt a) die Zugabe von 0,85 bis 1,0 Mol der Verbindung mit der Formel III innerhalb von 3,5 bis 4,5 Stunden bei 83 bis 87°C erfolgt, anschliessend während 1,4 bis 1,6 Stunden auf 33 bis 37°C abgekühlt wird, worauf die Zugabe von 1,45 bis 1,3 Mol der Verbindung mit der Formel III innerhalb von 5,5 bis 6,5 Stunden im Temperaturbereich von 33 bis 37°C erfolgt.

7. Verfahren gemäss Anspruch 1, worin in den Verfahrensschritten a) und b) das gleiche chlorierte aromatische Lösungsmittel eingesetzt wird.

8. Verfahren gemäss Anspruch 7, worin in den Verfahrensschritten a) und b) Chlorbenzol oder Dichlorbenzol eingesetzt wird.

9. Verfahren gemäss Anspruch 1, worin in Verfahrensschritt a) die Lewis-Säure in einem Verhältnis von 2,4 bis 2,6 Mol pro Mol Cyanurchlorid eingesetzt wird.

10. Verfahren gemäss Anspruch 9, worin die Lewis-Säure Aluminiumtrichlorid oder Aluminiumtribromid ist.

11. Verfahren gemäss Anspruch 10, worin die Lewis-Säure Aluminiumtrichlorid ist.

12. Verfahren gemäss Anspruch 1, worin in Verfahrensschritt b) 0,7 bis 0,8 Mol Resorcin pro Mol Cyanurchlorid eingesetzt werden.

13. Verfahren gemäss Anspruch 1, worin die Temperatur in Verfahrensschritt b) 35 bis 80°C beträgt.

14. Verfahren zur Herstellung von Verbindung der Formel IV 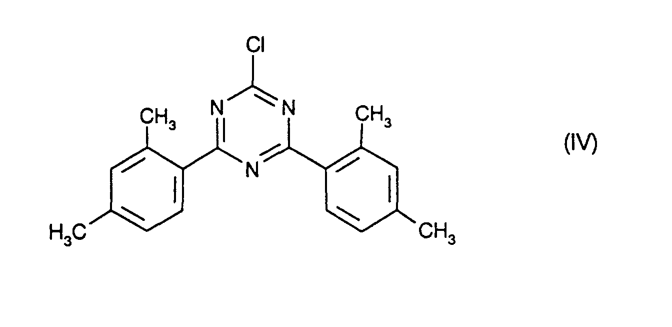 **dadurch gekennzeichnet, dass** man Cyanurchlorid der Formel II 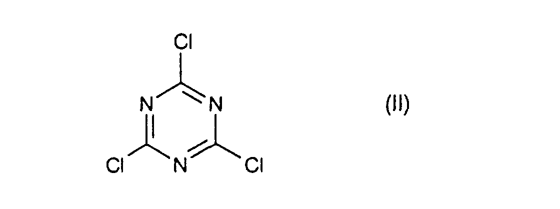 bei einer Temperatur von 0 bis 110°C in Gegenwart einer Lewis-Säure bei einem Verhältnis von 2,3 bis 3,0 Mol Lewis-Säure pro Mol eingesetztem Cyanurchlorid in Anwesenheit mindestens eines inerten chlorierten aromatischen Lösungsmittels mit 1,3-Xylol der Formel III 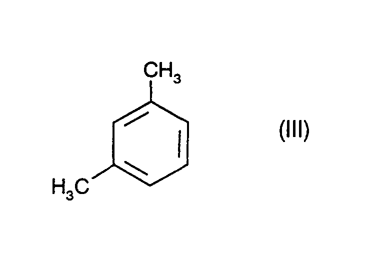 bei einem Verhältnis von 2,1 bis 2,5 Mol Verbindung der Formel III pro Mol eingesetztem Cyanurchlorid unter Zugabe von Verbindung der Formel III in einem Zeitraum von 5 bis 30 Stunden zur Verbindung der Formel IV umsetzt und die erhaltene Verbindung der Formel IV isoliert.

## Claims

1. A process for the preparation of 2-(2,4-dihydroxyphenyl)-4,6-bis-(2,4-dimethylphenyl)-s-triazine of the formula I 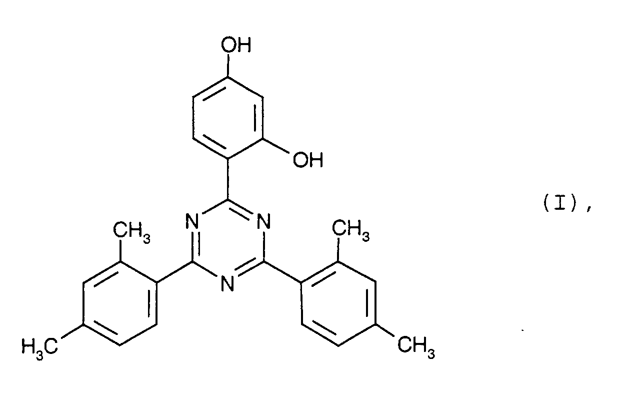 which comprises
a) reacting cyanuric chloride of the formula II 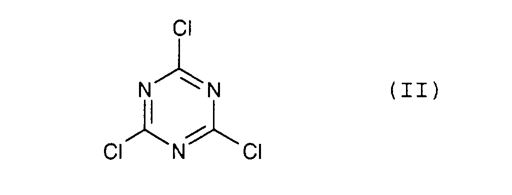 at a temperature of 0 to 110°C in the presence of a Lewis acid in a ratio of 2.3 to 3.0 mol of Lewis acid per mole of cyanuric chloride employed in the presence of at least one inert chlorinated aromatic solvent, with 1,3-xylene of the formula III 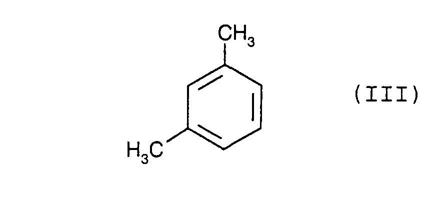 in a ratio of 2.1 to 2.5 mol of compound of the formula III per mole of cyanuric chloride employed with addition of compound of the formula III in a period of from 5 to 30 hours to give the compound of the formula IV 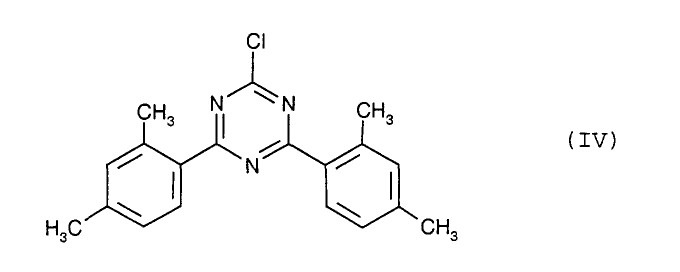 and then
b) without isolating the compound of the formula IV obtained reacting this with resorcinol in a ratio of 0.5 to 1.0 mol of resorcinol per mole of cyanuric chloride employed at a temperature from 0 to 100°C to give the compound of the formula I.

2. A process according to claim 1, wherein in process step a) the compound of the formula III is added in 2 portions.

3. A process according to claim 2, wherein first 0.5 to 1.1 mol and then 2.0 to 1.0 mol of the compound of the formula III are added.

4. A process according to claim 2, wherein the addition of the first portion is carried out at 60 to 110°C, that of the second at 20 to 50°C.

5. A process according to claim 1, wherein in process step a) the addition of 0.5 to 1.1 mol of the compound of the formula III at 60 to 110°C in the course of 2 to 12 hours, then the mixture is cooled to 20 to 50°C in the course of 0 to 3 hours, after which the addition of 2.0 to 1.0 mol of the compound of the formula III takes place in the temperature range from 20 to 50°C in the course of 5 to 15 hours.

6. A process according to claim 1, wherein in process step a) the addition of 0.85 to 1.0 mol of the compound of the formula III takes place at 83 to 87°C in the course of 3.5 to 4.5 hours, then the mixture is cooled to 33 to 37°C in the course of 1.4 to 1.6 hours, after which the addition of 1.45 to 1.3 mol of the compound of the formula III takes place in the temperature range from 33 to 37°C in the course of 5.5 to 6.5 hours.

7. A process according to claim 1, wherein in process steps a) and b) the same chlorinated aromatic solvent is employed.

8. A process according to claim 7, wherein in process steps a) and b) chlorobenzene or dichlorobenzene are employed.

9. A process according to claim 1, wherein in process step a) the Lewis acid is employed in a ratio of 2.4 to 2.6 mol per mole of cyanuric chloride.

10. A process according to claim 9, wherein the Lewis acid is aluminium trichloride or aluminium tribromide.

11. A process according to claim 10, wherein the Lewis acid is aluminium trichloride.

12. A process according to claim 1, wherein in process step b) 0.7 to 0.8 mol of resorcinol are employed per mole of cyanuric chloride.

13. A process according to claim 1, wherein the temperature in process step b) is 35 to 80°C.

14. A process for the preparation of the compound of the formula IV 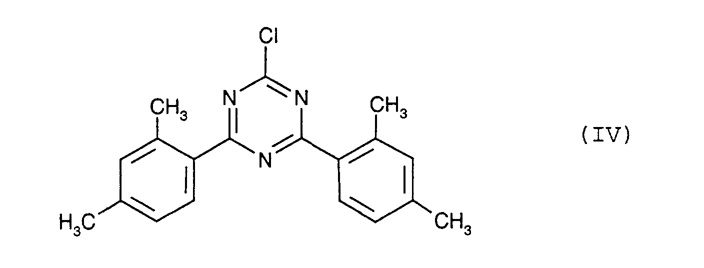 wherein cyanuric chloride of the formula II 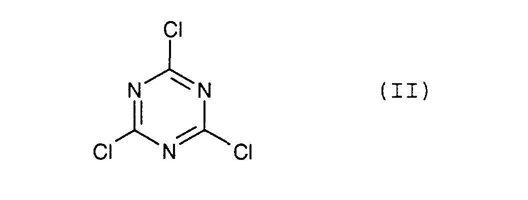 is reacted at a temperature of 0 to 110°C in the presence of a Lewis acid in a ratio of 2.3 to 3.0 mol of Lewis acid per mole of cyanuric chloride employed in the presence of at least one inert chlorinated aromatic solvent, with 1,3-xylene of the formula III 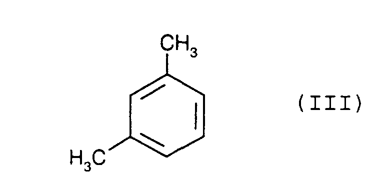 in a ratio of 2.1 to 2.5 mol of compound of the formula III per mole of cyanuric chloride employed with addition of compound of the formula III in a period of 5 to 30 hours to give the compound of the formula IV, and the compound of the formula IV obtained is isolated.

## Revendications

1. Procédé de préparation de la 2-(2,4-dihydroxyphényl)-4,6-bis(2,4-diméthylphényl)-s-triazine de formule I 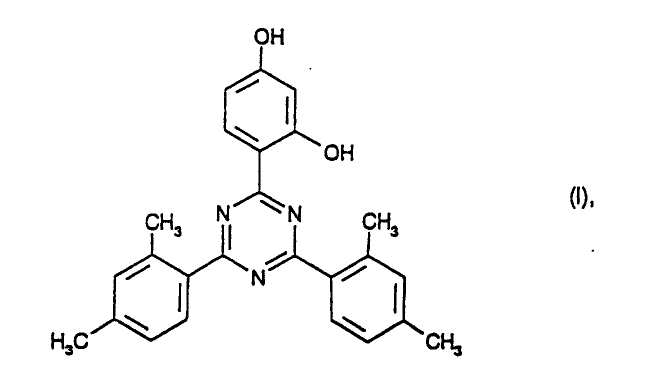 **caractérisé en ce que**
a) on fait réagir du chlorure de cyanuryle de formule II 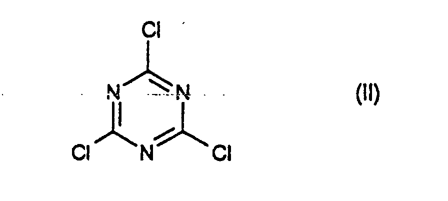 à une température de 0 à 110°C, en présence d'un acide de Lewis dans des proportions de 2,3 à 3,0 mol d'acide de Lewis par mol de chlorure de cyanuryle utilisé, en présence d'au moins un solvant aromatique chloré inerte, avec du 1,3-xylène de formule III 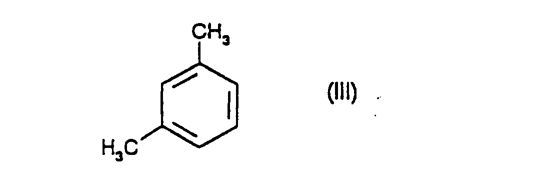 dans des proportions de 2,1 à 2,5 mol de composé de formule III par mol de chlorure de cyanuryle utilisé, en ajoutant le composé de formule III en l'espace de 5 à 30 heures, pour obtenir le composé de formule IV 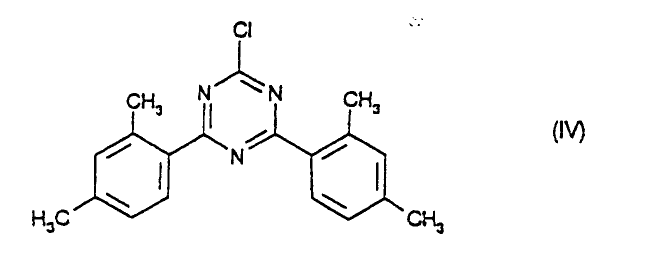 puis
b) sans isoler le composé de formule IV obtenu, on le fait réagir avec du résorcinol dans des proportions de 0,5 à 1,0 mol de résorcinol par mol de chlorure de cyanuryle utilisé, à une température de 0 à 100°C, pour obtenir le composé de formule I.

2. Procédé selon la revendication 1, dans lequel, dans l'étape de réaction a), on ajoute le composé de formule III en 2 portions.

3. Procédé selon la revendication 2, dans lequel on ajoute d'abord 0,5 à 1,1 mol, puis 2,0 à 1,0 mol du composé de formule III.

4. Procédé selon la revendication 2, dans lequel l'addition de la première portion s'effectue à une température de 60 à 110°C, et celle de la deuxième à une température de 20 à 50°C.

5. Procédé selon la revendication 1, dans lequel, dans l'étape de réaction a), on ajoute 0,5 à 1,1 mol du composé de formule III en l'espace de 2 à 12 heures à une température de 60 à 110°C, puis on refroidit à une température de 20 à 50°C en l'espace de 0 à 3 heures, après quoi on ajoute 2,0 à 1,0 mol du composé de formule III en l'espace de 5 à 15 heures dans l'intervalle de température de 20 à 50°C.

6. Procédé selon la revendication 1, dans lequel, dans l'étape de réaction a), on ajoute 0,85 à 1,0 mol du composé de formule III en l'espace de 3,5 à 4,5 heures à une température de 83 à 87°C, puis on refroidit à une température de 33 à 37°C en l'espace de 1,4 à 1,6 heure, après quoi on ajoute 1,45 à 1,3 mol du composé de formule III en l'espace de 5,5 à 6,5 heures dans l'intervalle de température de 33 à 37°C.

7. Procédé selon la revendication 1, dans lequel on utilise le même solvant aromatique chloré dans les étapes de réaction a) et b).

8. Procédé selon la revendication 7, dans lequel on utilise du chlorobenzène ou un dichlorobenzène dans les étapes de réaction a) et b).

9. Procédé selon la revendication 1, dans lequel, dans l'étape de réaction a), on utilise l'acide de Lewis en des proportions de 2,4 à 2,6 mol par mol de chlorure de cyanuryle.

10. Procédé selon la revendication 9, dans lequel l'acide de Lewis est le trichlorure d'aluminium ou le tribromure d'aluminium.

11. Procédé selon la revendication 10, dans lequel l'acide de Lewis est le trichlorure d'aluminium.

12. Procédé selon la revendication 1, dans lequel, dans l'étape de réaction b), on utilise 0,7 à 0,8 mol de résorcinol par mol de chlorure de cyanuryle.

13. Procédé selon la revendication 1, dans lequel la température dans l'étape de réaction b) est de 35 à 80°C.

14. Procédé de préparation du composé de formule IV 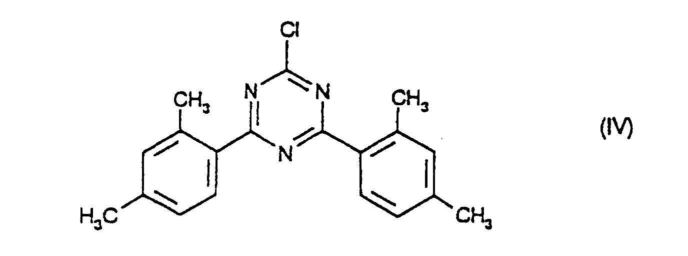 **caractérisé en ce que** l'on fait réagir du chlorure de cyanuryle de formule II 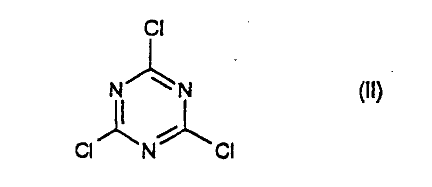 à une température de 0 à 110°C, en présence d'un acide de Lewis dans des proportions de 2,3 à 3,0 mol d'acide de Lewis par mol de chlorure de cyanuryle utilisé, en présence d'au moins un solvant aromatique chloré inerte, avec du 1,3-xylène de formule III 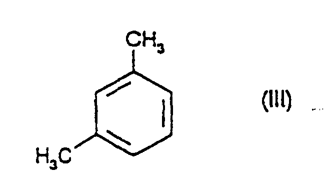 dans des proportions de 2,1 à 2,5 mol de composé de formule III par mol de chlorure de cyanuryle utilisé, en ajoutant le composé de formule III en l'espace de 5 à 30 heures, pour obtenir le composé de formule IV, et on isole le composé de formule IV obtenu.
